(19) **Europäisches Patentamt**

**European Patent Office**

**Office européen des brevets**

(11) **EP 0 982 284 A2**

(12) **EUROPÄISCHE PATENTANMELDUNG**

(43) Veröffentlichungstag:
   **01.03.2000 Patentblatt 2000/09**

(51) Int. Cl.$^7$: **C07C 29/76**, C07C 31/18,
   B01D 61/42

(21) Anmeldenummer: **99115961.7**

(22) Anmeldetag: **13.08.1999**

(84) Benannte Vertragsstaaten:
   **AT BE CH CY DE DK ES FI FR GB GR IE IT LI LU MC NL PT SE**
   Benannte Erstreckungsstaaten:
   **AL LT LV MK RO SI**

(30) Priorität: **24.08.1998 DE 19838426**

(71) Anmelder:
   • **Degussa-Hüls Aktiengesellschaft**
   **60311 Frankfurt am Main (DE)**
   Benannte Vertragsstaaten:
   **BE CH DE DK ES FI FR GB GR IE IT NL PT SE AT**

   • **Servo Delden B.V.**
   **7491 AE  Delden (NL)**
   Benannte Vertragsstaaten:
   **BE CH DE DK ES FI FR GB GR IE IT NL PT SE AT**

(72) Erfinder:
   • **Hörpel, Gerhard, Dr.**
   **48301 Nottuln (DE)**
   • **Jakob, Antje, Dr.**
   **27283 Verden/Aller (DE)**
   • **Mangnus, Eddy, Dr.**
   **7422 NV Deventer (NL)**
   • **van der Velden, Paulus Martinus, Dr.**
   **7555 MK Hengelo (NL)**

(54) **Verfahren zum Trennen von Trimethylolpropan von wasserlöslichen Salzen aus einer Lösung**

(57)   Die Erfindung betrifft ein Verfahren zum Trennen von Trimethylolpropan von wasserlöslichen Salzen aus einer Lösung unter Verwendung eines Elektrodialyseverfahrens.

**EP 0 982 284 A2**

**Beschreibung**

Die vorliegende Erfindung betrifft ein Verfahren zum Trennen von Trimethylolpropan von wasserlöslichen Salzen aus einer Lösung.

Trimethylolpropan ist ein handelsüblich erhältliches Produkt und wird z.B. als Wärmeübertragungsmittel, als Gefrierschutzmittel, als Ausgangsmaterial bei der Herstellung von Kunstharzen (z.B. Polyurethanen), bei der Herstellung von Additiven für die Lackindustrie (Coatings), als Weichmacher für Kunststoffe und als Schmier- und Gleitmittel (Lubricants) verwendet.

Trimethylolpropan wird z.B. industriell durch basisch katalysierte Reaktion (z.B. in Gegenwart von Kalilauge oder Natronlauge) von Butyraldehyd mit Formaldehyd hergestellt. Bei dieser Umsetzung entsteht in stöchiometrischen Mengen das Alkalisalz der Ameisensäure (z.B. Kaliumformiat oder Natriumformiat). Da die Alkali- und Erdalkalisalze der niederhomologen organischen Säuren im allgemeinen gut bis sehr gut wasserlöslich sind, lassen sie sich nicht ohne weiteres von dem ebenfalls gut wasserlöslichen Polyol (Triol) abtrennen. Erschwerend kommt hinzu, daß auch die reinen Stoffe eine mehr oder weniger gute Löslichkeit ineinander haben.

Es sind verschiedene Verfahren zur Extraktion von Polyolen sowie Verfahren zum Auskristallisieren der Salze vorgeschlagen worden, um die wasserlöslichen Polyole von den ebenfalls wasserlöslichen Salzen abzutrennen.

Bei der Extraktion mit Lösungsmitteln für die Polyole, wie z.B. mit Amylalkohol, Cyclohexanol oder verschiedenen Estern, sind große Mengen an Extraktionsmittel erforderlich, die anschließend wieder z.B. durch Destillation vom gewünschten Produkt abgetrennt werden müssen.

Die US-PS 2468718 beschreibt ein Verfahren zum Trennen von Methylolalkanen von wasserlöslichen Salzen durch Extraktion der Methylolalkane mit einem niedrigsiedenden, wasserlöslichen Keton.

In Ullmann, Band 7, 4. Auflage, S. 231 (1974) wird beschrieben, daß das Gemisch aus Polyolen und Salzen durch Extraktion mit Lösungsmitteln, wie Amylalkohol, Cyclohexanol oder Essigester, und anschließender Destillation der Polyole aufgearbeitet werden kann. Diese Extraktionsverfahren erfordern jedoch die Verwendung großer Mengen an Extraktionsmitteln, die nachfolgend entfernt werden müssen. Dabei werden intensiv gefärbte Produkte erhalten, die eine Vielzahl von Nebenprodukten enthalten und die hauptsächlich wegen ihrer nachteiligen Farbe einer weiteren Destillation unterworfen werden müssen.

Die Abtrennung der Nebenprodukte ist deshalb von Bedeutung, weil diese in den nachfolgenden Anwendungen zu nicht beherrschbaren Störungen führen würden. So beeinflussen gefärbte Nebenprodukte in der Lackanwendung die Reproduzierbarkeit der Coatings nachteilig. Darüberhinaus wirken sich Reste von Salzen störend bei der Umsetzung des Trimethylolpropans in nachfolgenden Reaktionen aus, z.B. bei der Herstellung von Trimethylolpropanestern. Aber auch jene Nebenprodukte, die sich in nachfolgenden Umsetzungen des Trimethylolpropans umsetzen lassen, beeinflussen die Eigenschaften der Trimethylolpropanumsetzungsprodukte, wie z.B. die Viskosität, nachteilig.

Es ist ebenfalls möglich, das Gemisch aus Polyolen und Salzen aufzukonzentrieren und die Salze auszukristallisieren (CN-A-1076185). Aber auch bei diesem Verfahren werden gefärbte Produkte erhalten. Hierzu kommt, daß im Kristallisat beträchtliche Mengen an Polyolen verbleiben, die sich nur unter deutlichen Ausbeuteverlusten auswaschen lassen.

Aufgabe der vorliegenden Erfindung ist es, ein verbessertes Verfahren zum Trennen von Trimethylolpropan von wasserlöslichen Salzen aus einer Lösung bereitzustellen, das kostengünstig, d.h. mit geringem Energieverbrauch, durchgeführt werden kann, das ohne Einsatz von Lösungsmitteln auskommt und somit umweltfreundlich durchgeführt werden kann, und mit dem ein farbloses Triol erhalten wird, das ohne weitere Reinigungsprozesse direkt weiterverarbeitet werden kann. Der Ausdruck "Triol" umfaßt im folgenden Trimethylolpropan (TMP) mit OH-funktionellen Nebenprodukten, wie z.B. Di-TMP.

Diese Aufgabe wird erfindungsgemäß durch ein Verfahren zum Trennen von Trimethylolpropan von wasserlöslichen Salzen aus einer Lösung gelöst, das dadurch gekennzeichnet ist, daß als Trennverfahren ein Elektrodialyseverfahren verwendet wird.

Die Verwendung der Elektrodialyse zur Meerwasserentsalzung, zur Solegewinnung, zur Trinkwassergewinnung, zur Regulierung des Härtegrades von Wasser, zur Molkeentsalzung in der Lebensmittelindustrie, zur Verhinderung der Weinsteinabscheidung bei der Weinherstellung oder zur Rückgewinnung wertvoller Stoffe aus galvanischen Abwässern ist bekannt (Römpp Chemie Lexikon, Band 2, 10. Auflage, S. 1113-1114 (1997)). Bei diesen Anwendungen handelt es sich jedoch immer um wäßrige Systeme mit einem relativ geringen Salzgehalt (< 5 Gew.%).

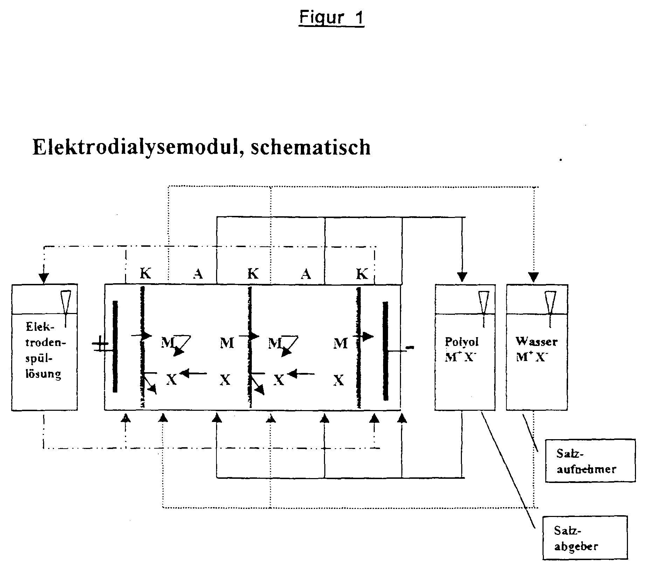

Die Elektrodialyse ist ein Trennverfahren, bei dem die Wanderung von Ionen durch permselektive Membranen mit einer angelegten elektrischen Gleichspannung beschleunigt wird. Bei der Elektrodialyse werden Ionen unter der Einwirkung eines elektrischen Feldes durch Membranen transportiert. Wenn in der Dialysevorrichtung die Ionenaustauschermembranen so angeordnet werden, daß sich abwechselnd je eine Anionenaustauschermembran und eine Kationenaustauschermembran zwischen zwei Elektroden befinden und die Zelle in schmale Kammern teilen, so werden bei entsprechender Schaltung salzärmere und salzreichere Volumenströme erhalten, denn bei Stromdurchgang können die Kationen nur die Kationenaustauschermembranen passieren und die Anionen nur die Anionenaustauschermembranen. Die Anreicherung erfolgt gegen den Konzentrationsgradienten. Das heißt, durch Hintereinanderschalten mehrerer anionen- und kationenselektiver Ionenaustauschermembranen läßt sich die Entionisierung der zu dialysierenden Flüssigkeit bei gleichzeitiger Anreicherung der Ionen in den Elektrodenzellen erreichen. Eine schematische Ansicht einer Elektrodialysevorrichtung ist in Figur 1 gezeigt.

Es wurde nun überraschenderweise gefunden, daß die Elektrodialyse ebenfalls dazu verwendet werden kann, um ein wäßriges Gemisch von Trimethylolpropan und wasserlöslichen Salzen aufzutrennen, selbst wenn die Salze in hoher Konzentration (>50 Gew.-%) vorliegen. Dabei lassen sich hohe Selektivitäten (S > 250) erreichen.

Die Selektivität der Auftrennung zwischen der ursprünglich salzhaltigen und danach entsetzten Lösung sowie der konzentrierten Lösung bezüglich der neunten Komponente Polyol wird wie bei sonstigen Membranverfahren definiert:

$$S = ([Polyol]_{Konz}[Salz]_{Konz})/([Polyol]_{Dil}[Salz]_{Dil})$$

Dabei bedeuten:

Dil:             Salzabgeberlösung
Konz:          Salzaufnehmerlösung
[Polyol]:     Gewichtsanteil Polyol der Lösung
[Salz]:        Gewichtsanteil der Salze in der Lösung

Die Selektivität des Salztransports wird durch die Stromausbeute beschrieben, d.h. die tatsächlich transportierte Stoffmenge an Salz ($N_{gemessen}$ in Mol) bezogen auf die durch den elektrischen Ladungstransport (elektrischer Strom) maximale mögliche Menge ($N_{theoretisch}$).

$$\eta = N_{gemessen}/N_{theoretisch}$$

Bei der Entsalzung der Lösung des Triols kann trotz des hohen Salzgehaltes eine Stromausbeute von über 95% erreicht werden.

Die in dem erfindungsgemäßen Verfahren verwendete Elektrodialysevorrichtung besteht aus handelsüblich erhältlichen Elektrodialysemodulen mit ebenfalls handelsüblich erhält-. lichen Anionen- und Kationenaustauschermembranen. Hierbei muß gegebenenfalls die Anionenaustauschermembran nach den Kriterien a) geringer Widerstand, b) hohe Selektivität in Bezug auf das Anion und c) geringer Lösungsmittelfluß ausgesucht werden.

Die Elektrodialysevorrichtung kann einen Salzabgeberkreislauf und einen Salzaufnehmerkreislauf umfassen, wie in Figur 1 gezeigt.

Die Mischung aus Polyol und Salzen, wie sie z.B. als Rohprodukt aus der basenkatalysierten Aldoladdition des Butyraldehyds mit Formaldehyd und anschließender Cannizzaro-Reaktion in Gegenwart stöchiometrischer Mengen an Base (z.B. Alkalihydroxid) entsteht, kann unmittelbar dem Salzabgeberkreislauf einer Elektrodialysevorrichtung gemäß Figur 1 zugeführt werden. Es ist dabei unerheblich, wie hoch die Konzentration der Einzelkomponenten in der Mischung ist, solange die Mischung pumpbar ist.

Der pH-Wert der Salzabgeberlösung wird mit einer Säure, bevorzugt mit Ameisensäure, oder einer Base, bevorzugt mit der gleichen Base, die bei der Herstellung des Triols verwendet wurde, z.B. mit Alkalihydroxid, annähernd neutral eingestellt. Saure bzw. alkalische pH-Werte sind möglich, solange die Stabilität der Membranen hiervon nicht berührt wird. Bevorzugt sind pH-Werte im Bereich von 4 bis 10.

Die Obergrenze der Temperatur der Salzabgeberlösung wird durch die Stabilität der Ionenaustauschermembranen bestimmt; die Untergrenze der Temperatur wird durch die Viskosität bzw. die Pumpbarkeit des Mediums bestimmt. Die Temperatur wird jedoch bevorzugt auf einen Wert im Bereich von 10 bis 50°C eingestellt. Dabei muß berücksichtigt werden, daß sich die Salzabgeberlösung während des Trennprozesses erwärmt.

Die Salzaufnehmerlösung in dem Salzaufnehmerkreislauf der Elektrodialysevorrichtung besteht bevorzugt aus Wasser oder einer wäßrigen Salzlösung. Der pH-Wert der Salzaufnehmerlösung wird vorzugsweise auf einen Wert im Bereich von 4 bis 10 eingestellt, und die Temperatur der Lösung liegt bevorzugt im Bereich von 10 bis 50°C. Auch in diesem Fall wird die Obergrenze der Konzentration des Mediums durch die Pumpbarkeit der Lösung bestimmt. Wichtig ist auch, daß das Löslichkeitsprodukt der durch die Membranen permeierenden Anionen und Kationen in der Salzaufnehmerlösung nicht überschritten wird. Salzablagerungen im Salzaufnehmerkreislauf können zu irreversiblen Schäden an der gesamten Vorrichtung, insbesondere an den Membranen führen.

Die Stromdichte bei der Elektrodialyse liegt bevorzugt im Bereich von 50 bis 750 A/m$^2$, besonders bevorzugt im Bereich von 150 bis 250 A/m$^2$. Die Stromausbeute kann unter optimalen Prozeßbedingungen mehr als 95% betragen. Die Grenzstromdichte muß an die Salzkonzentration in dem zu entsalzenden Gemisch angepaßt werden und kann durch den Fachmann leicht ermittelt werden. Die Grenzstromdichte als Funktion der Konzentration bestimmt somit die Regelung der Stromdichte während des Trennprozesses.

In der erfindungsgemäß verwendeten Elektrodialysevorrichtung werden bevorzugt Elektrodenspüllösungen eingesetzt, wie in Figur 1 gezeigt. Die Elektrodenspüllösungen stellen sicher, daß keine Reaktion der Elektroden mit den Stoffen der Lösung stattfindet, daß die bei der Elektrodenreaktion entstehenden Gase ausgetragen werden können, und daß durch eine hohe Leitfähigkeit der elektrische Widerstand verringert und damit der Energiebedarf der Elektrodenreaktion minimiert wird. Die Elektrodenspüllösung sollte, wenn möglich, die gleichen Kationen wie die anderen Salzlösungen enthalten, um ein Eindringen weiterer Kationen in den Prozeß zu vermeiden. Als Elektrodenspüllösungen können wäßrige Lösungen anorganischer Salze eingesetzt werden.

Der Verlauf des Trennprozesses kann über die Leitfähigkeit der Salzabgeber- und Salzaufnehmerlösung verfolgt werden. Die Leitfähigkeit muß zu den analytisch bestimmten Gehalten an Triol bzw. Salz korreliert werden, d.h., daß bei einer gegebenen Leitfähigkeit mit Hilfe anderer analytischer Methoden (z.B. Hochleistungsflüssigchromatographie, HPLC, Gaschromatographie, GC) die tatsächliche Konzentration an Trimethylolpropan, Nebenprodukten und Salz bestimmt werden muß. Im allgemeinen reicht es aus, die Elektrodialyse so lange durchzuführen, bis die Leitfähigkeit der Salzabgeberlösung auf etwa 2 μS/cm abgefallen ist. Die Reinigung des gesamten Elektrodialysemoduls hängt von der Trennaufgabe ab. Für die Reinigung des Systems ist es beispielsweise ausreichend, das Modul alle zwei Wochen etwa 2 Stunden lang mit warmem VE-Wasser zu spülen.

Bei der basisch katalysierten Reaktion (z.B. mit Kalilauge oder Natronlauge) von Butyraldehyd mit Formaldehyd entsteht Trimethylolpropan nicht als Reinsubstanz, sondern je nach Prozeßführung im Gemisch mit Di-DMP und weiteren OH-funktionellen Verbindungen sowie dem entsprechenden Formiatsalz aus der Cannizzaro-Reaktion. Wird dieses Rohprodukt mittels Elektrodialyse entsalzt, bleibt eine wäßrige, farblose Lösung des Polyolgemisches zurück, die als solche oder auch in aufkonzentrierter Form ohne weitere Reinigungsprozesse weiteren Reaktionen unterworfen werden kann, z.B. Kondensationsreaktionen, wie Veresterungen mit z.B. Ölsäure zur Herstellung von Lubricants, oder mit Acrylsäure zur Herstellung von Coatingadditiven.

Sollte dennoch das Trimethylolpropan aus dem Gemisch als Reinsubstanz isoliert werden, stehen die destillativen Stofftrennungsmethoden zur Verfügung.

Die folgenden Beispiele erläutern die Erfindung.

Beispiel 1:

Synthese des Triols, KOH-Route, kontinuierlich

Ein mit Stickstoff gespülter 100 ml Doppelwandkolben 1 wird auf +10°C gekühlt. Dieser Kolben ist ausgestattet mit 3 Dosiermöglichkeiten und einem Ausgang zu einem weiteren 500 ml Vakuumkolben 2. Der Ausgang des Kolbens 1 ist so angebracht, daß ein Flüssigkeitsniveau von 40 ml eingehalten werden kann. Über einen Zeitraum von 150 Minuten werden nun gleichzeitig 80 g frisch destillierter Butyraldehyd, 275,68 g Formalinlösung (38,34 Gew.-%) und 130,87 g KOH-Lösung (48,99 Gew.-%) hinzudosiert. Dabei wird nach ca. 15 Minuten das Flüssigkeitsniveau von 40 ml in Kolben 1 erreicht, so daß bei Anlegen eines leichten Vakuums das Reaktionsmedium kontinuierlich in den Kolben 2 gesaugt wird. Während des Prozesses wird die Kühlung so eingestellt, daß die Temperatur des Reaktionsmediums in Kolben 1 bei ca. 32°C gehalten werden kann.

Eine Nachbehandlung des gesamten Inhalts des Kolbens 2 über einen Zeitraum von 2,5 Stunden bei 85°C ergibt nach Zufügen von 3,6 g Wasserstoffperoxid und anschließendem Neutralisieren mit 2,4 g KOH Trimethylolpropan (TMP) in einer Ausbeute von 90% und Kaliumformiat in einer Ausbeute von 100%. Neben dem TMP werden noch höherkondensierte Alkohole identifiziert, von denen der größte Anteil mit 9% auf Di-TMP entfällt.

Beispiel 2:

Synthese des Triols, NaOH-Route, kontinuierlich

Ein mit Stickstoff gespülter 100 ml Doppelwandkolben 1 wird auf +10°C gekühlt. Dieser Kolben ist ausgestattet mit 3 Dosiermöglichkeiten und einem Ausgang zu einem weiteren 500 ml Vakuumkolben 2. Der Ausgang des Kolbens 1 ist so angebracht, daß ein Flüssigkeitsniveau von 40 ml eingehalten werden kann. Über einen Zeitraum von 150 Minuten werden nun gleichzeitig 80 g frisch destillierter Butyraldehyd, 276,33 g Formalinlösung (38,34 Gew.-%) und 92,02 g NaOH-Lösung (49,67 Gew.-%) hinzudosiert. Dabei wird nach ca. 15 Minuten das Flüssigkeitsniveau von 40 ml in Kolben 1 erreicht, so daß bei Anlegen eines leichten Vakuums das Reaktionsmedium kontinuierlich in den Kolben 2 gesaugt wird. Während des Prozesses wird die Kühlung so eingestellt, daß die Temperatur des Reaktionsmediums in Kolben 1 bei ca. 32°C gehalten werden kann.

Eine Nachbehandlung des gesamten Inhalts des Kolbens 2 über einen Zeitraum von 2,5 Stunden bei 85°C ergibt nach Zufügen von 3,6 g Wasserstoffperoxid und anschließendem Neutralisieren mit 2,51 g NaOH Trimethylolpropan (TMP) in einer Ausbeute von 90% und Natriumformiat in einer Ausbeute von 100%. Neben dem TMP werden noch höherkondensierte Alkohole identifiziert, von denen der größe Anteil mit 9% auf Di-TMP entfällt.

Beispiel 3:

Trennung der Rohlösung aus Beispiel 1

In einem Labormodul mit einer effektiven Membranfläche von 100 cm$^2$ wurde die Reaktionslösung, die gemäß Beispiel 1 hergestellt worden war, bestehend aus 26,7 Gew.-% Kaliumformiat, 33,9 Gew.-% (Trimethylolpropan + höherkondensierte Alkohole) und Wasser, durch Elektrodialyse entsalzt. Der Versuchsaufbau bestand aus fünf Zeiteinheiten sowie der Anoden- und Kathodenkammer. Im Konzentrat wurde eine schwach verdünnte, rein wäßrige Kaliumformiatlösung vorgelegt. Im Anodenkreis befand sich eine Kaliumcarbonatlösung. Als Membranen wurden Neosepta AHA2 und C66 10F von Tokuyama Soda eingesetzt.

Während des Versuchs würde die Stromstärke aufgrund der abnehmenden Leitfähigkeit im Entsalzungskreislauf von 2,73 A auf 0,3 A abfallen. Zur Aufrechterhaltung der Stromstärke wurde deshalb die angelegte Spannung von 29 V (zu Beginn des Versuchs) auf über 80 V (am Ende des Versuchs) erhöht.

Nach einer Versuchstaufzeit von 11 Stunden befanden sich im Diluatkreislauf 65,0 Gew.-% Triol und 0,03 Gew.-% Kaliumformiat, im Konzentratkreislauf 0,2 Gew.-% Triol und 27,4 Gew.-% Kaliumformiat.

Beispiel 4:

Trennung einer Testlösung

In einem weiteren Versuch wurde im Labor in einem größeren Testmodul mit einer effektiven Membranfläche von 375 cm$^2$ ein synthetisches Gemisch aus 33 Gew.-% Kaliumformiat, 44 Gew.-% TMP und dem Rest Wasser entsatzt. Der Stapelaufbau bestand aus drei Zeiteinheiten sowie den Anoden- und Kathodenkammern. Während des Versuchs würde die Stromstärke aufgrund der abnehmenden Leitfähigkeit im Entsalzungskreislauf von 8 A auf 1 A abfallen. Zur Aufrechterhaltung der Stromstärke wurde deshalb die angelegte Spannung von 10 V (zu Beginn des Versuchs) auf 80 V (am Ende des Versuchs) erhöht. Zur Trennung wurden Neosepta AHA2 und C66 10F Membranen von Tokuyama Soda verwendet, im Konzentratkreislauf wurde eine verdünnte Kaliumformiatlösung, im Anodenkreis eine Kaliumcarbonatlösung vorgelegt.

Auch bei diesem Versuch konnte nahezu alles Kaliumformiat aus dem Entsalzungskreis (von 32,3 Gew.-% auf 0,022 Gew.-%) abgetrennt werden. Die Stromausbeute bei dieser Entsalzung betrug 89,1%. Im Verlauf des Versuchs wurden zudem ca. 10 g TMP als neutrale Teilchen mit über die Membranen transportiert. Dies bedeutet, bezogen auf die Ausgangsmenge, einen TMP-Verlust von ca. 1,95 Gew.-%.

Die genauen Konzentrationswerte können den nachfolgenden Tabellen entnommen werden.

Tabelle 1

| Elektrodialysedaten zu Beispiel 4: | | **Salzabgeber** | | **Salzaufnehmer** | |
|---|---|---|---|---|---|
| | | Anfang | Ende | Anfang | Ende |
| Kaliumformiat | g | 401,6 | 0,15 | 0,3 | 398,9 |
| | % | 32,2 | 0,022 | 0,02 | 21,5 |
| TMP | g | 527,2 | 487,39 | 0,08 | 10,3 |
| | % | 42,3 | 69,453 | 0,006 | 0,5 |
| H$_2$O | g | 315,6 | 214,21 | 1383,7 | 1444,9 |
| | % | 25,3 | 30,525 | 99,9 | 77,9 |
| Gesamt | g | 1244,4 | 436,98 | 1384,1 | 1698,8 |

Beispiel 5:

Synthese des Triols, KOH-Route, kontinuierlich, pilot scale

In einem Kreisstromreaktor mit einem Kreis-/Austragsverhältnis von 10:1 werden 46 Liter enthärtetes Trinkwasser vorgelegt. Danach werden gleichzeitig drei getrennte Dosierströme gestartet von Butyraldehyd mit 6,0 kg/Stunde, Formaldehydlösung (37,6 Gew.-%) mit 21,0 kg/Stunde und Kalilauge (50 Gew.-%) mit 9,3 kg/Stunde. Mittels einer Wasserkühleinrichtung wird die Starttemperatur zwischen 17°C und 19°C gehalten, und die Reaktionstemperatur während der 4,5-stündigen Reaktion wird bei ≤ 29°C gehalten. 192 kg des so angefallenen Triols werden unter Stickstoff und Rühren auf 70°C bis 75°C aufgeheizt und 2,5 Stunden lang bei dieser Temperatur nachgerührt. Nach Zugabe von 20 kg Wasserstoffperoxid (40 Gew.-%) wurde die Reaktionsmischung über Nacht abgekühlt, wobei der pH-Wert neutral war (7,2).

Beispiel 6:

Trennung der Rohlösung aus Beispiel 5

In einem 4-Kammer-Elektrodialysemodul (4 x 100 l) mit Kathodenkreislauf (verdünnte Kaliumformiatlösung) und Anodenkreislauf (Kaliumsulfatlösung) sowie Diluat- und Konzentratkreislauf wurde eine Menge Rohlösung bzw. Wasser gemäß der folgenden Tabelle vorgelegt. Beim Öffnen der Pumpenkreisläufe wurde sichergestellt, daß der Druckunterschied diesseits und jenseits der Membranen (Neosepta AHA2 und C66 10F Membranen von Tokuyama Soda) nicht mehr als 0,2 bar betrug. Alle Kreisläufe wurden mit einem Druck von 0,7 bis 0,8 bar und einer Fließgeschwindigkeit von 400 l/Stunde betrieben. Die Spannung betrug maximal 40 V. Die Temperatur stieg auf maximal 45°C. Innerhalb von 20 Stunden wurde im Diluat bis zu einer Leitfähigkeit von 2 mS/cm gefahren.

Tabelle 2

| Elektrodialysedaten zu Beispiel 6: | | | | | |
|---|---|---|---|---|---|
| | | Salzabgeber | | Salzaufnehmer | |
| | | Anfang | Ende | Anfang | Ende |
| Kaliumformiat | kg | 11,91 | 0,077 | 0,03 | 10,84 |
| | % | 17,0 | 0,19 | 0,075 | 16,2 |
| TMP | kg | 7,84 | 7,84 | 0,008 | 0,528 |
| | % | 11,2 | 19,6 | 0,02 | 0,8 |
| $H_2O$ | kg | 45,92 | 30,53 | 39,96 | 55,0 |
| | % | 65,6 | 75,95 | 99,9 | 82,3 |
| Gesamt | kg | 70,0 | 40,2 | 40,0 | 66,8 |

Beispiel 7:

Veresterung des Triols aus Beispiel 6 mit Ölsäure

612 g Triol aus Beispiel 6 (120 g trocken) werden zunächst über einen Filmverdampfer im Vakuum bei einer Bodentemperatur von maximal 90°C entwässert. Der zurückbleibende farblose, viskose Stoff wird mit 658,8 g Rapsölsäure (Molmasse 280) versetzt und im $N_2$-Strom aufgeheizt. Nach 2 Stunden werden bei einer Bodentemperatur von 210°C 37,4 g Destillat, nach 3,5 Stunden bei einer Bodentemperatur von 270°C 45,3 g Destillat und nach weiteren 0,5 Stunden bei einer Bodentemperatur von 280°C und einem Vakuum von 30 mbar insgesamt 49,8 g Destillat aufgefangen. Im Reaktionsgefäß befinden sich 726,9 g Ölsäuretriolester als klare, leicht braune Flüssigkeit.

Beispiel 8:

Veresterung des Triols aus Beispiel 6 mit Acrylsäure

1224 g Triol aus Beispiel 6 (240 g trocken) werden wie in Beispiel 7 entwässert. Das zurückbleibende Triol wird mit 373 g Acrylsäure und 0,414 g Hydrochinonmonomethylether in 207,2 g Toluol gelöst. Nach Zugabe von 8,3 g Toluolsulfonsäure-monohydrat und Durchleiten von Luft und Aufheizen wird azeotrop Wasser abdestilliert. Nach 8 Stunden entstanden 86,5 g Destillatwasser. Das Rohprodukt des Triesters wird als toluolische Lösung zweimal alkalisch und zweimal neutral gewaschen und anschließend die toluolische Phase vom Lösungsmittel destillativ befreit. Es wurden 591 g Trioltriacrylat erhalten.

## Patentansprüche

1. Verfahren zum Trennen von Trimethylolpropan von wasserlöslichen Salzen aus einer Lösung, **dadurch gekennzeichnet**, daß als Trennverfahren ein Elektrodialyseverfahren verwendet wird.

2. Verfahren nach Anspruch 1, **dadurch gekennzeichnet**, daß Trimethylolpropan als Rohprodukt aus einer Umsetzung von Butyraldehyd, Formaldehyd und einem Alkalihydroxid stammt und von weiteren OH-funktionellen Nebenprodukten begleitet ist.

3. Verfahren nach Anspruch 1 oder 2, **dadurch gekennzeichnet,** daß das Elektrodialyseverfahren bei einer Temperaturvon 10°C bis 50°C durchgeführt wird.

4. Verfahren nach Anspruch 1, 2 oder 3, **dadurch gekennzeichnet,** daß das Elektrodialyseverfahren bei einem pH-Wert von 4 bis 10 durchgeführt wird.

5. Verfahren nach Anspruch 4, **dadurch gekennzeichnet,** daß das Elektrodialyseverfahren bei einem pH-Wert von etwa 7 durchgeführt wird.

6. Verfahren nach mindestens einem der Ansprüche 1 bis 5, **dadurch gekennzeichnet,** daß das Elektrodialyseverfahren bei einer Stromdichte von 50 bis 750 A/m$^2$ durchgeführt wird.

7. Verwendung der nach mindestens einem Verfahren der Ansprüche 1 bis 6 erhaltenen Triole zur Weiterverarbeitung als Ester zur Herstellung von Performancechemikalien wie z.B. Additive für Coatings.

## Figur 1

**Elektrodialysemodul, schematisch**